Europäisches Patentamt

European Patent Office

Office européen des brevets

(11) Publication number : **0 456 402 A1**

## (12) EUROPEAN PATENT APPLICATION

(21) Application number : **91303938.4**

(22) Date of filing : **01.05.91**

(51) Int. Cl.⁵ : **B08B 9/02, A46B 9/02**

(30) Priority : **09.05.90 JP 47660/90**

(43) Date of publication of application :
**13.11.91 Bulletin 91/46**

(84) Designated Contracting States :
**DE FR GB**

(71) Applicant : **KABUSHIKI KAISHA MACHIDA SEISAKUSHO**
**13-8, Honkomagome 6-chome Bunkyo-ku Tokyo (JP)**

(72) Inventor : **Yoshizawa, Hidetoshi**
**39-11, Ikebukurohoncho 2-Chome, Toshima-Ku**
**Tokyo (JP)**
Inventor : **Miyagi, Kunihiko**
**27-17, Honcho**
**Wako-Shi, Saitama-Ken (JP)**

(74) Representative : **Votier, Sidney David**
**CARPMAELS & RANSFORD 43, Bloomsbury Square**
**London WC1A 2RA (GB)**

(54) Cleaning brush.

(57)    A brush for cleaning an inner surface of a tube (13) includes an elongated support member (1), and a number of bristles (2 ; 2A) mounted on the support member, each of the bristles extending in a direction intersecting an axis of said support member. The number of bristles constitute a brush portion (3 ; 3A). The brush portion has a portion where the lengths of the bristles from the support member are decreasing progressively in the direction of the axis of the support member. When the brush is inserted into the tube, part of the bristles are slightly bent and strongly pressed against the inner surface of the tube. When the brush is moved along the tube, the thus bent bristles strongly rub the inner surface of the tube.

Fig.5

EP 0 456 402 A1

BACKGROUND OF THE INVENTION

This invention relates to a brush for cleaning an inner surface of a tube such as a forceps guide tube of an endoscope.

An endoscope is provided with a tube for guiding a forceps and for drawing a body fluid. Once the endoscope is used, cells, a body fluid, various germs (bacteria) and etc., adhere to the inner surface of this tube. Therefore, before the endoscope is used again, the inner surface of the tube is cleaned by a cleaning brush as disclosed in Japanese Laid-Open (Kokai) Utility Model Application No. 180009/86.

The above cleaning brush has an elongated operating member so flexible as to be bent, a wire member fixedly secured to the distal end of the operating member and extending forwardly therefrom, and a number of bristles mounted on the wire member. The wire member is composed of two metal wire elements twisted together. The bristles have the same length, and are juxtaposed along the length of the wire member in generally perpendicular relation to the wire member. The central portions of the bristles are fixedly held between the two metal wire elements. Since the two wire elements are twisted, any two adjacent bristles are displaced with respect to each other in the direction of the periphery of the wire member. Namely, one ends of the juxtaposed bristles are disposed on a spiral line, and the other ends of the bristles are also disposed on a spiral line. In the above prior art publication, for the sake of simplicity of illustration, the bristles are shown in a common plane.

For cleaning the above tube, there is selected the brush having the bristles whose length is slightly greater than the inner diameter of the tube. As a result of selecting such a brush, the bristles, when inserted into the tube, are slightly bent in such a manner that the opposite ends of the bristles are strongly pressed against the inner surface of the tube. This provides an enhanced cleaning effect. If there is used the brush having the bristles whose length is considerably greater than the inner diameter of the tube, the bristles are bent to a greater extent, so that their opposite ends can only be held in contact with the inner surface of the tube under a weak force. In this case, the cleaning effect is naturally low. If there is used the brush having the bristles whose length is smaller than the inner diameter of the tube, the opposite ends of the bristles can not be satisfactorily contacted with the inner surface of the tube, in which case the cleaning effect is very low.

Conventionally, there are prepared a plurality of kinds of brushes having respective bristles of different lengths, and a suitable one of the brushes is selected depending on the diameter of the tube to be cleaned. In this case, it is cumbersome to select a proper one out of the plurality of brushes, and also it is possible to select a wrong brush. Further, much space must be provided for storing the plurality of brushes.

U. S. Patent No. 4,889,106 and Japanese Laid-Open Utility Model Application Nos. 116201/82 and 116202/82 disclose brushes for insertion into an endoscope to collect cells. These brushes are identical in basic construction to the above-mentioned cleaning brush. In these publications, the shape of a brush portion having a number of bristles of the same length is shown more clearly. Namely, the bristles are slightly displaced with respect to one another in the direction of the periphery of the brush portion, and the opposite ends of the bristles are disposed on two spiral lines, respectively. Therefore, when viewed in a direction perpendicular to a wire member of the brush, the brush portion is seen as corrugated.

SUMMARY OF THE INVENTION

It is an object of this invention to provide a cleaning brush which can satisfactorily clean an inner surface of any of a plurality of tubes of different diameters.

According to the present invention, there is provided a brush for cleaning an inner surface of a tube, comprising:

(a) an elongated support member; and
(b) a number of bristles mounted on the support member and each extending in a direction intersecting an axis of the support member, the number of bristles constituting a brush portion, and the brush portion having a portion where the lengths of the bristles from the support member are decreasing progressively in the direction of the axis of the support member.

BRIEF DESCRIPTION OF THE DRAWINGS

Fig. 1 is a plan view of a wire-bristles assembly provided at a first step of a method of producing a cleaning brush of the present invention, showing a number of juxtaposed bristles of the same length as held between a pair of wire elements;
Fig. 2 Is a front-elevational view of the wire-bristles assembly of Fig. 1 with the pair of wire elements twisted;
Fig. 3 is an enlarged, front-elevational view of a portion of the wire-bristles assembly of Fig. 2;
Fig. 4 is a front-elevational view of the wire-bristles assembly of Fig. 3 with the bristles cut;
Fig. 5 is a front-elevational view of the cleaning brush in its final form;
Fig. 6 is a front-elevational view of an endoscope to be cleaned by the cleaning brush of Fig. 5;
Fig. 7 is a view showing the bristles as indicated in a common plane;
Fig. 8 is a front-elevational view of the distal end portion of the cleaning brush in a cleaning condition; and

Fig. 9 is a front-elevational view of a distal end portion of a modified cleaning brush.

## DESCRIPTION OF PREFERRED EMBODIMENTS OF THE INVENTION

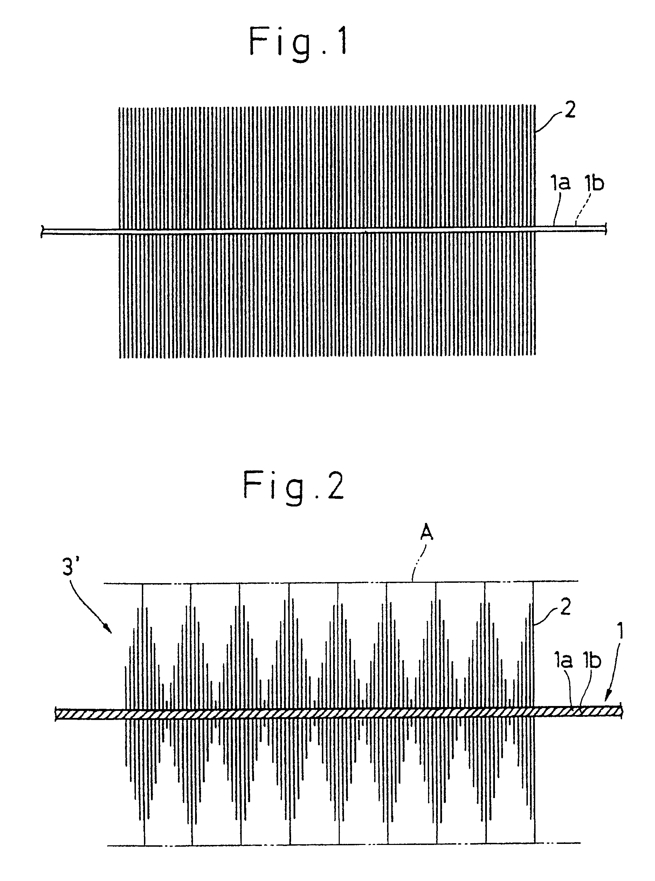

One preferred embodiment of the invention will now be described with reference to the drawings. First, a method of producing a cleaning brush according to the present invention will be described. As shown in Fig. 1, a number of bristles 2 of nylon having the same length are placed between two wire elements 1a and 1b of stainless steel disposed parallel to each other and spaced from each other in a direction perpendicular to the sheet of Fig. 1. The bristles 2 are juxtaposed along the length of the wire elements 1a and 1b, and are disposed in contact with one another. The bristles 2 are disposed perpendicular to the wire elements 1a and 1b, and the central portions of the bristles 2 are interposed between the wire elements 1a and 1b. The bristles 2 are indicated by lines in Fig. 1.

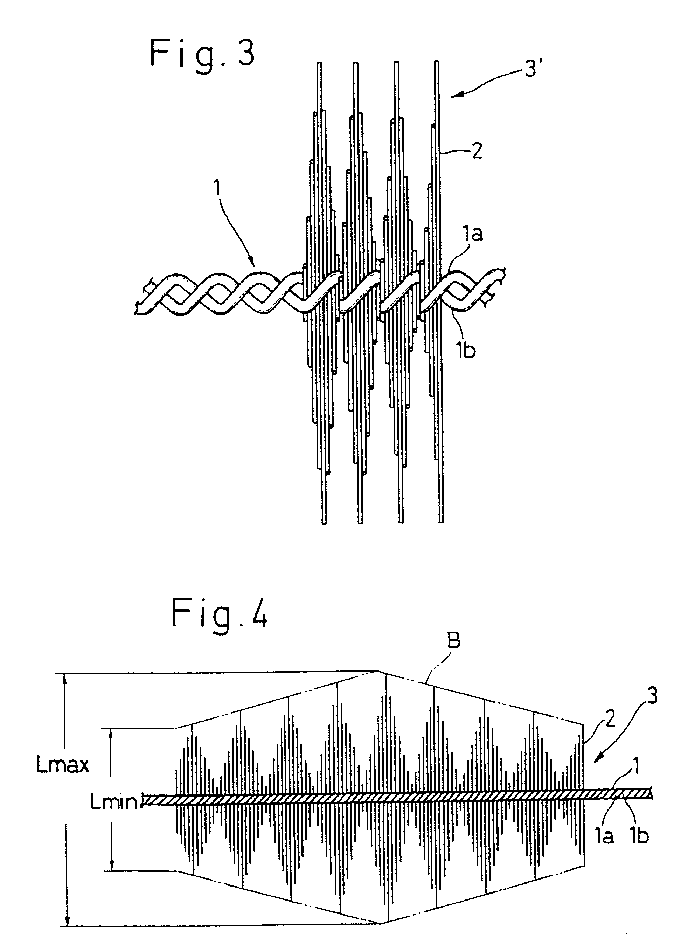

Then, the wire elements 1a and 1b are twisted together while they are tensioned or pulled. As a result, a wire member (elongated support member) 1 composed of the twisted wire elements 1a and 1b is provided, as shown in Figs. 2 and 3. The central portions of the bristles 2 are firmly held between the twisted wire elements 1a and 1b, so that the bristles 2 are fixedly mounted on the wire member 1. Although for example, 30 to 40 bristles 2 are provided per pitch of the wire elements 1a and 1b, a smaller number of bristles 2 are shown in Figs. 2 and 3 for the sake of simplicity of the illustration.

The above steps are the same as those of the conventional method, and a brush portion 3' formed by a number of bristles 2 of the same length has the same shape as that of the conventional brush. Namely, the bristles 2 extend generally perpendicular to the axis of the wire member 1. The juxtaposed bristles 2 are slightly displaced with respect to one another in the direction of the periphery of the wire member 1, and one ends of the bristles 2 are disposed on a spiral line whereas the other ends of the bristles 2 are also disposed on a spiral line. The diameters of these two spiral lines are equal to each other, and are uniform in the longitudinal direction of the wire member 1. In other words, the opposite ends of the bristles 2 of the brush portion 3' are all disposed on an imaginary cylindrical surface A having its center disposed at the axis of the wire member 1.

Then, the bristles 2 are cut in a manner shown in Fig. 4 to provide a brush portion 3 of a final shape. That bristle 2 disposed at the central portion of the brush portion 3 has the maximum length Lmax, and the lengths of the bristles 2 are decreasing progressively linearly at a constant rate toward the opposite ends of the brush portion 3. Those bristles 2 disposed respectively at the opposite ends of the brush portion 3 have the same length, that is, the minimum length Lmin. One ends of the juxtaposed bristles 2 are disposed on a spiral line, and this spiral line has the maximum diameter at the central portion of the brush portion 3, and is decreasing in diameter progressively toward the opposite ends of the brush portion 3. Similarly, the other ends of the juxtaposed bristles 2 are disposed on a spiral line. In other words, the opposite ends of the bristles 2 of the brush portion 3 are all disposed on an imaginary generally-cylindrical surface B. This cylindrical surface B has the maximum diameter at the central portion of the brush portion 3, and is decreasing in diameter progressively toward the opposite ends of the brush portion 3, so that the cylindrical surface B has the minimum diameter at each of the opposite ends of the brush portion 3.

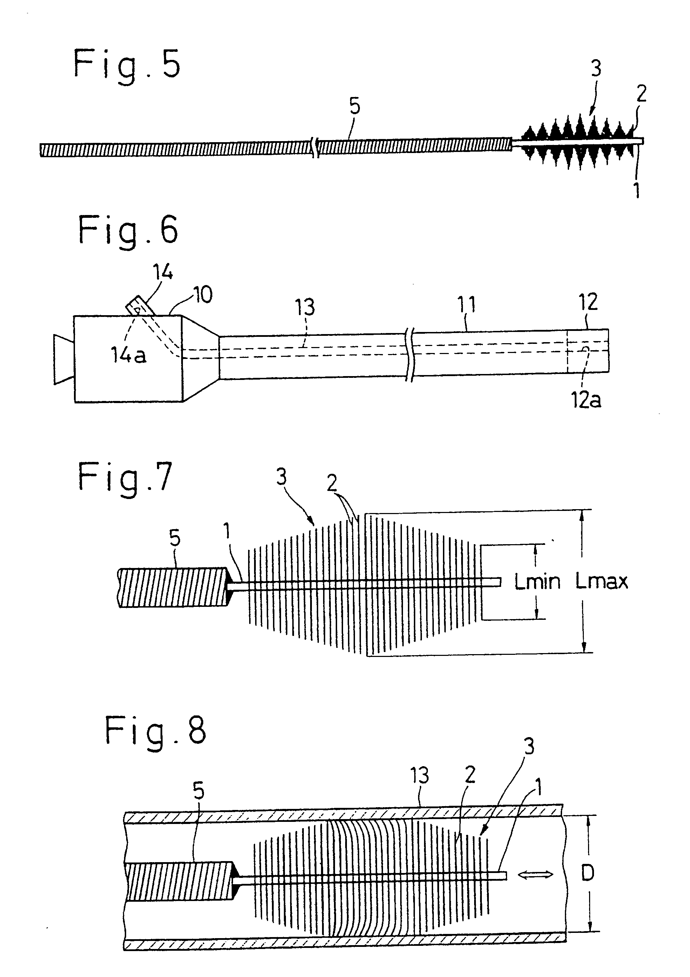

Then, the opposite end portions of the wire member 1 are cut off to bring the wire member 1 into a predetermined length. Then, as shown in Fig. 5, one end portion of the wire member 1 is fixedly secured to an elongated operating member 5, thereby providing the cleaning brush (final product). The operating member 5 is made of a stainless steel wire which is spirally wound into a tubular shape. The operating member 5 of this construction is so flexible as to be bent. The one end portion of the wire member 1 is inserted into the distal end portion of the operating member 5, and is fixed thereto by soldering or the like.

Fig. 6 shows an endoscope to be cleaned by the cleaning brush of the above construction. This endoscope is well known, and therefore will be described briefly. The endoscope comprises a tubular body 10, an insertion tube 11 extending from the front end of the body 10, and a rigid tip 12 mounted on the distal end of the insertion tube 11. The insertion tube 11 is so flexible as to be bent. A tube 13, as well as optical fiber bundles and other parts, is received in the insertion tube 11. The proximal end of the tube 13 is connected to a hole 14a formed in a projection 14 formed on the body 10, and the distal end of the tube 13 is connected to a hole 12a formed in the tip 12. The hole 14a of the projection 14, the hole 12a of the tip 12 and the internal space (bore) of the tube 13 jointly constitute a channel which is used to guide a forceps and/or to draw a body liquid. When the holes 12a and 14a and the tube 13 are used as such a guide channel or such a suction channel, the inner surface of the tube 13 is contaminated with cells, a body liquid and etc.

The cleaning of the tube 13 of the endoscope by the use of the cleaning brush of the above construction will now be described with reference to Figs. 7 and 8. In these Figures, the bristles 2 are shown in a common plane for the purpose of the better understanding of this cleaning operation. A requirement for the cleaning operation is that the inner diameter D of the tube 13 should be in a predetermined range. More specifically, the inner diameter D of the tube 13 is smaller than a value obtained by subtracting a predetermined value from the maximum diameter Lmax of the brush portion 3. Also, the inner diameter D of the tube 13 is greater than a value obtained by subtracting a predetermined value from the minimum diameter Lmin of the brush portion 3.

The cleaning brush is inserted into the tube 13 through the hole 14a in the projection 14 of the endoscope, and the operating member 5 is reciprocally moved (that is, pushed and pulled) in its longitudinal direction, so that the brush portion 3 is reciprocally moved along the tube 13. At this time, those bristles 2 whose length is smaller than the inner diameter D of the tube 13 hardly contribute to the cleaning of the inner surface of the tube 13. However, those bristles 2 whose length is slightly greater than the inner diameter D of the tube 13 are slightly bent, and are strongly pressed at their opposite ends against the inner surface of the tube 13 to rub the same, thereby efficiently removing or separating cells, a body liquid and etc., adhering to the inner surface of the tube 13. Those bristles 2 which are much longer are bent considerably, and remove cells, weakly adhering to the inner surface of the tube 13, in a sweeping manner.

For cleaning a tube (having a diameter different from that of the above-mentioned tube 13) of another endoscope, if the inner diameter of the tube meets the above-mentioned requirement, a satisfactory cleaning effect can be obtained since part of bristles 2 having different lengths strongly rub the inner surface of the tube. Thus, the tubes of a plurality kinds of endoscopes can be cleaned with one cleaning brush.

Since the brush portion 3 is decreasing in diameter toward its distal end, the brush portion 3 can be easily inserted into the hole 14a of the projection 14. Further, since the brush portion 3 is also decreasing in diameter toward its proximal end, the brush portion 3 can be easily brought back into the hole 12a of the tip 12 after the brush portion 3 is passed forwardly through the hole 12a.

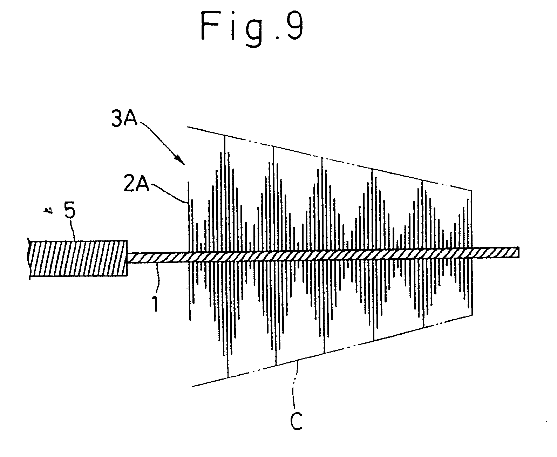

Fig. 9 shows a modified cleaning brush of the invention. Those portions of this brush corresponding respectively to those of the cleaning brush of the preceding embodiment are denoted by identical reference numerals, and detailed explanation thereof will be omitted. A brush portion 3A of this embodiment is different in shape from the brush portion 3 of the preceding embodiment. More specifically, that bristle 2A disposed at the proximal end (left end in Fig. 9) of the brush portion 3A has the maximum length, and the lengths of the bristles 2A are decreasing progressively linearly toward the distal end of the brush portion 3A, so that that bristle 2A disposed at the distal end of the brush portion 3A has the minimum length. In other words, an imaginary cylindrical surface C on which the opposite ends of the bristles 2A are all disposed has the maximum diameter at the proximal end of the brush portion 3A, and is decreasing in diameter progressively toward the distal end of the brush portion 3A.

The present invention is not limited to the above embodiments, and various modifications can be made. For example, the brush of the present invention is not only used for cleaning the tube of the endoscope, but also may be used for cleaning a tube incorporated in a catheter. Also, the brush of the present invention may be used for cleaning a bellows-like tube, in which case the inner surfaces of the greater-diameter portions of the tube are strongly rubbed by the longer bristles, and the inner surfaces of the smaller-diameter portions are strongly rubbed by the shorter bristles, thereby achieving a good cleaning effect.

## Claims

1. A brush for cleaning an inner surface of a tube, comprising:
   (a) an elongated support member; and
   (b) a number of bristles mounted on said support member and each extending in a direction intersecting an axis of said support member, said number of bristles constituting a brush portion; CHARACTERIZED in that:
   said brush portion (3; 3A) has a portion where the lengths of said bristles (2; 2A) from said support member (1) are decreasing progressively in the direction of the axis of said support member.

2. A brush according to claim 1, in which that bristle (2) disposed at a position intermediate opposite ends of said brush portion (3) has the maximum length, the lengths of said bristles being decreasing progressively toward the opposite ends of said brush portion.

3. A brush according to claim 2, in which that bristle (2) disposed at the center of said brush portion (3) in the axial direction of said brush portion has the maximum length, the lengths of said bristles being decreasing progressively at a constant rate toward the opposite ends of said brush, so that said bristles disposed respectively at the opposite ends of said brush portion are equal in length to each other, and has the minimum length.

4. A brush according to claim 1, in which that bristle (2A) disposed at one end of said brush portion (3A) has the maximum length, and the lengths of said bristles being decreasing progressively toward the other end of said brush portion, so that said bristle disposed at said other end of said brush portion has the minimum length.

5. A brush according to claim 1, in which said elongated support member (1) is a wire member composed of two metal wire elements (1a, 1b) twisted together, each of said number of bristles (2) being held at its central portion between said two twisted wire elements, and said number of bristles extending generally perpendicular to the axis of said support member and being juxtaposed in the direction of the axis of said support member.

6. A brush according to claim 5, further comprising an elongated operating member (5) so flexible as to be bent, one end of said support member (1) being fixedly secured to a front end of said operating member.

# Fig.1

# Fig.2

# Fig. 3

# Fig. 4

# Fig.5

# Fig.6

# Fig.7

# Fig.8

# Fig.9

## EUROPEAN SEARCH REPORT

**European Patent Office**

Application Number

EP 91 30 3938

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | US-A-4819291 (GUNJIAN) <br> * column 1, line 65 - column 2, line 54; figures 1-3 * | 1, 4-6 | B08B9/02 <br> A46B9/02 |
| Y | DE-A-2648108 (NIEDERER) <br> * page 6, line 1 - page 7, line 21; figures 1-3 * | 1, 4-6 | |
| A | DE-B-1032961 (HEMPEL) <br> * claims 1, 3, 5; figure 3 * | 1, 2 | |
| A | GB-A-682455 (COLES (CONTRACTORS) LTD) <br> * claim 1; figure 3 * | 1 | |
| A | GB-A-2159420 (MEDSCAND) <br> * abstract; figure 2 * | 1 | |
| A | DE-C-579919 (BRENDLIN) <br> * claim 1; figures 1-4 * | 1 | |
| | | | TECHNICAL FIELDS SEARCHED (Int. Cl.5 ) <br><br> A46B <br> B08B <br> A61B |

The present search report has been drawn up for all claims

| Place of search | Date of completion of the search | Examiner |
|---|---|---|
| THE HAGUE | 13 AUGUST 1991 | ERNST R.T. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)